# EUROPÄISCHE PATENTSCHRIFT

(11) **EP 2 055 315 B1**
(45) Veröffentlichungstag und Bekanntmachung des Hinweises auf die Patenterteilung: **16.03.2011**
(21) Anmeldenummer: 08019125.7
(22) Anmeldetag: 31.10.2008
(51) Int. Cl.: A61K 47/32, A61K 8/81, A61Q 19/00, A61Q 5/02, C08F 2/32

(54) **Verdickungsmittel und dessen Anwendung**
Thickening agent and its application
Agent épaississant et son utilisation

(30) Priorität: 02.11.2007 DE 102007052864
(43) Veröffentlichungstag der Anmeldung: 06.05.2009
(73) Patentinhaber: Worlée-Chemie G.m.b.H., 22113 Hamburg (DE)
(72) Erfinder: Von Eben-Worlée, Reinhold, 22297 Hamburg (DE); Woelk, Kristin, 23626 Ratekau (DE); Brune, Dirk, 23617 Stockelsdorf (DE); Dahms, Gerd Herbert, 47138 Duisburg (DE); Jung, Andreas Heinrich, Dr., 47198 Duisburg (DE)
(74) Vertreter: UEXKÜLL & STOLBERG

(56) Entgegenhaltungen:
- EP-A- 0 214 758
- EP-A- 1 166 771
- DE-A1- 10 051 351
- US-A1- 2006 223 945
- 'paraffin', [Online] Gefunden im Internet: <URL:http://de.wikipedia.org/wiki/Paraffin> [gefunden am 2009-12-30]

## Beschreibung

Die vorliegende Erfindung betrifft eine Zusammensetzung, die als Verdickungsmittel, insbesondere in kosmetischen Formulierungen, verwendet werden kann und ein Verfahren zu ihrer Herstellung.

Es ist im Stand der Technik bekannt, dass thermodynamisch an sich instabile kolloide Systeme, wie Dispersionen, Emulsionen, Suspensionen, Schäume, die z. B. in der kosmetischen und pharmazeutischen Industrie in einer Vielzahl zur Anwendung kommen, stabilisiert werden müssen, um ein Absetzen zu verhindern, Dispergierhilfen zu geben, Emulsionen oder Schäume zu bilden. Diese Aufgaben können durch ein geeignetes Verdickungsmittel übernommen werden. Zahlreiche Verdickungsmittel sind bekannt, und können in folgende Gruppen eingeteilt werden:
- Organische, natürliche Verdickungsmittel wie z. B. Agar-Agar, Carrageen, Tragant, Gummi arabicum, Alginate, Pektione, Polyosen, Guar-Mehl, Johannisbrotkernmehl, Stärke, Dextrine, Gelatine, Casein
- Organisch abgewandelte Naturstoffe wie Carboxymethylcellulose, Celluloseether, Hydroxyethyl- u. -propylcellulose, Kernmehlether
- Organisch vollsynthetische Verdickungsmittel wie Polyacrylu. Polymethacrylverbindungen, Vinylpolymere, Polycarbonsäuren, Polyether, Polyimine, Polyamide.
- Anorganische Verdickungsmittel wie Polykieselsäuren, Tonmineralien, Zeolithe, Kieselsäuren.

Diese Verdickungsmittel sind aber in der kosmetischen und pharmazeutischen Industrie nicht ohne Nachteile zu verwenden. So variieren die natürlichen Produkte in Qualität und Farbe, und führen weiterhin zu Produkten mit begrenzter Haltbarkeit. Da es sich überwiegend um Feststoffe handelt ist sehr darauf zu achten, dass es nicht zu Zusammenballungen und Klumpenbildungen kommt, und dass die Stoffe überhaupt in dem gewünschten Medium löslich bzw. quellfähig sind. Viele synthetische Produkte als auch die anorganischen Produkte benötigen bestimmte pH-Bereiche um wirkungsvoll zu sein.

Stand der Technik sind ferner Verdickungsmittel auf Basis synthetischer Polymere, die insbesondere von Acrylsäurederivaten abgeleitet sind. So beschreibt die DE 699 19 414 T2 (EP 1 233 750 B1) ein Verdickungsmittel, das als polymeren Bestandteil einen verzweigten oder vernetzten anionischen Polyelektrolyten auf Basis von teilweise oder vollständig in die Salzform überführter, mit Acrylamid copolymerisierter 2-Methyl-2,1-oxo-2-propylamino-1-propansulfonsäure (nachstehend kurz "AMPS") enthält, und deren Ölphase aus Isohexadecan besteht.

Die EP-A-0 503 853 offenbart ein Verdickungsmittel, das durch inverse Emulsionspolymerisation hergestellt wird und dessen polymerer Bestandteil sich von den Monomeren Acrylamid, AMPS, die teilweise als Salz vorliegt, und einem polyfunktionellen Monomer (Vernetzungsmittel) ableitet.

Die US-A-2001/051686 offenbart eine Zusammensetzung, die ebenfalls als Verdickungsmittel verwendet werden kann, das in Form eines sogenannten selbstinvertierbaren inversen Latex vorliegt, der als polymeren Bestandteil einen Polyelektrolyten enthält, wobei der Polyelektrolyt entweder ein Homopolymer auf Basis eines Monomers, das eine funktionelle Gruppe einer starken Säure aufweist, die teilweise oder vollständig als Salz vorliegt, oder ein Copolymer auf Basis des vorstehend erwähnten Monomers copolymerisiert mit Acrylamid sein kann. Die Ölphase der Zusammensetzung ist aus den Bestandteilen Mineralöl, Squalan, hydriertem Polyisobuten, Isohexadecan und Isododecan ausgewählt. Als Emulgatoren werden Sorbitanester wie beispielsweise Sorbitanmonooleat (Wasser-in-Öl-Emulgator, nachstehend auch kurz "W/O-Emulgator") und ethoxylierte Sorbitanester, z. B. Sorbitanoleat, das mit 20 Molen Ethylenoxid etoxyliert ist (Öl-in-Wasser-Emulgator, nachstehend auch kurz "O/W-Emulgator"), verwendet.

Die DE 695 23 022 T2 (= EP 0 716 594 B1) offenbart ein Verdickungsmittel auf Basis einer invertierten Wasser-in-Öl-Emulsion (nachstehend auch kurz "W/O-Emulsion"), in der die Wasserphase ein Copolymer mit von Acrylsäure in freier Form oder in Form des Natriumsalzes und von einem Monoacrylamidmonomer abgeleiteten Einheiten enthält. Die Ölphase besteht aus einem Gemisch eines flüchtigen und eines nicht-flüchtigen Öls. Als Beispiele für flüchtige Öle sind Isoparaffine genannt. Als nicht-flüchtige Öle sind allgemein Paraffinöle und durch Kondensation von Fettsäuren mit Alkoholen erhaltene Ester mit mehr als 10 Kohlenstoffatomen genannt. Als Beispiel für den letztgenannten Bestandteil ist Isostearylisostearat genannt.

Das US-Patent Nr. 6,197,287 B1 offenbart ein Verdickungsmittel auf Basis eines Copolymers aus AMPS in teilweise oder vollständig neutralisierter Form sowie einem weiteren Monomer mit einer vergleichsweise schwächeren Säurefunktion oder einem neutralen Monomer. Als Beispiele für schwach saure Monomere werden Acrylsäure, Methacrylsäure, Itakonsäure und Maleinsäure genannt. Als Beispiele für neutrale Monomere werden 2-Hydroxyethylacrylat, 2,3-Dihydroxypropylacrylat, 2-Hydroxyethylmethacrylat und 2,3-Dihydroxypropylmethacrylat oder ethoxylierte Derivate davon genannt. Als Ölphasen in den offenbarten Verdickungsmitteln kommen eine Reihe von Alternativen wie beispielsweise Paraffine, Isoparaffine oder Cycloparaffine in Betracht. Als Emulgatoren werden die schon vorstehend genannten Sorbitanester bzw. deren ethoxylierte Derivate verwendet.

Die DE 699 15 802 T2 offenbart ein polymeres Verdickungsmittel, das nach einem Verfahren hergestellt wird, bei dem eine wässrige Lösung aus einem Monomer mit starker Säurefunktion, die auf einen pH-Wert von unter 4 eingestellt wurde, in Gegenwart eines W/O-Emulgators in einer Ölphase emulgiert wird, das Monomer dann durch Einbringen eines Radikalstarters polymerisiert wird und nach Beendigung der Polymerisation bei einer Temperatur von unter 50 °C ein O/W-Emulgator eingearbeitet wird, wobei in Abwesenheit von Monoacrylamid gearbeitet wird. Als Emulgatoren finden beispielsweise Sorbitanmonooleat und ethoxylierte Sorbitanester Verwendung.

Die EP-A-0 186 361 offenbart ein Verdickungsmittel zum Verdicken von wässrigen Medien, welches Verdickungsmittel eine Wasser-in-Öl-Emulsion umfasst und als polymeren Bestandteil ein vernetztes Polymer enthält, das von einem ungesättigten Monomer abgeleitet ist, das eine ionisierbare, vorzugsweise anionische Gruppe aufweist. Als geeignete anionische Monomere sind Acrylsäure, Methacrylsäure, deren Alkali- oder Ammoniumsalze, AMPS, 2-Sulfoethylmethacrylat, Styrolphosphonsäure, Natriumstyrolsulfonat, Phosphonsäurederivate von Acrylat- oder Methacrylatestern sowie Mischungen dieser Monomere genannt. Das Verdickungsmittel ist insbesondere für die Verwendung in Druckpasten gedacht. Die Öl-phase des Verdickungsmittels beruht auf Isoparaffinen oder Silikonölen.

Die EP-A-1 166 771 betrifft eine unter anderem als Verdickungsmittel verwendbare Zusammensetzung in Form eines inversen Latex, der eine Ölphase, die neben Fettsäureester größere Mengen Isoparaffin enthält, eine wässrige Phase, mindestens einen W/O-Emulgator, mindestens einen O/W-Emulgator und einen verzweigten oder vernetzten Polyelektrolyten enthält.

Die DE 100 51 351 A1 betrifft kosmetische Emulsionen, die einen Ölkörper, Emulgatoren sowie Polymere vom Latextyp enthalten, wobei die Latices ausschließlich durch Kondensation von Monomeren mit starken Säurefunktionen und Co-Monomeren erhalten werden, welche entweder eine Säurefunktion aufweisen oder neutral reagieren.

Die EP-A-0 214 758 betrifft ein Polymerisationsverfahren, bei dem eine Wasser-in-Öl-Emulsion hergestellt wird, in deren wässriger Phase Acrylamid- oder Acrylsäurehomopolymer oder Copolymer gelöst ist, wobei ein spezielles Tensid verwendet wird, das eine gesättigte oder ungesättigte Kohlenwasserstoffkette mit 30 bis 500 Kohlenstoffatomen und einen endständigen Bernsteinsäureanhydridrest umfasst, wobei dieser Rest eine hydrophile Gruppe trägt. Die Ölphase besteht aus paraffinischen Kohlenwasserstoffen.

Die US-A-2006/223945 betrifft Synthesetechniken zur Herstellung von Emulgatoren mit schwacher Farbe aus Maleinsäureanhydrid, Polymeren von Isobutylen.

Zusammengefasst werden in den obigen Dokumenten des Standes der Technik Wasser-in-Öl-Emulsionen beschrieben, die insbesondere auf vernetzten Copolymeren von Acrylamid und 2-Acrylamido-2-methylpropansulfonsäure (AMPS) beruhen und in synthetischen Ölen dispergiert sind. Insbesondere wird ferner Isohexadecan oder ein Isoparaffin als kontinuierliche Öl-Phase bzw. signifikante Bestandteile davon verwendet.

Der Erfindung liegt somit die Aufgabe zu Grunde, eine Zusammensetzung und ein Verfahren zur Herstellung derselben bereitzustellen, die als Verdickungsmittel insbesondere für kosmetische und pharmazeutische Formulierungen geeignet ist, und Nachteile der aus dem Stand der Technik bekannten Verdickungsmittel, z. B. die Verwendung gesundheitlich bedenklicher Stoffe wie Acrylamid, vermeidet. Das erfindungsgemäße Verdickungsmittel sollte reproduzierbar mit gleichbleibender Qualität herstellbar sein und sich leicht in kosmetische und pharmazeutische Formulierungen einarbeiten lassen.

Die obige Aufgabe wird durch ein Verdickungsmittel gelöst, das auf einer Zusammensetzung basiert, die eine Ölphase, eine wässrige Phase und mindestens einen Wasser-in-Öl-Emulgator (W/O-Emulgator) enthält, wobei die wässrige Phase mindestens einen verzweigten oder vernetzten anionischen Polyelektrolyten enthält, der durch Copolymerisation von 2-Acrylamido-2-methylpropansulfonsäuresalz (AMPS-Salz) mit teilweise neutralisierter Acrylsäure herstellbar ist, und die Ölphäse aus mindestens einem Ester eines C₁- bis C₅-Alkohols und einer gesättigten oder ungesättigten C₁₀- bis C₂₂-Fettsäure besteht.

Ferner stellt die vorliegende Erfindung eine Emulgatorkombination zur Verfügung, die aus mindestens einem W/O-Emulgator und mindestens einem O/W-Emulgator besteht und die dadurch gekennzeichnet, dass der W/O-Emulgator ein Bernsteinsäurederivat ausgewählt aus der Gruppe bestehend aus stickstoffhaltigen Polyisobutenyl-substituierten Bernsteinsäureestern, -halbestern, -salzen, -halbsalzen, Mischformen dieser Verbindungen und Mischungen davon ist und der O/W-Emulgator ausgewählt ist aus der Gruppe bestehend aus ethoxylierten primären oder sekundären C₁₀- bis C₂₀-Alkoholen und anionischen Sulfosuccinaten sowie Mischungen davon. Ferner wird auf die Definition der Emulgatoren in den abhängigen Ansprüchen Bezug genommen, insbesondere Ansprüche 5 bis 9 und 10 bis 11.

Eine bevorzugte Emulgatorzusammensetzung umfasst als W/O-Emulgator Hydroxyethyldiethonium Polyisobutenyl Triethylaminosuccinat und als O/W-Emulgator ein Polyethylenglycolether einer Mischung von sekundären C₁₁- bis C₁₅-Fettalkoholen mit durchschnittlich 9 Molen Ethylenoxid oder ein anionisches Sulfosuccinat.

Die vorliegende Erfindung stellt ferner einen Polyelektrolyten zur Verfügung, der durch Copolymerisation von einem AMPS-Salz, insbesondere dem Natriumsalz, und Acrylsäure in teilweise neutralisierter Form herstellbar ist, wobei das Verhältnis der vom AMPS-Salz abgeleiteten Einheiten zu den von Acrylsäure in teilweise neutralisierter Form abgeleiteten Einheiten im Bereich von 5:1 bis 0,5:1 liegt, und der mit 0,2-2 Millimol Verzweigungsmittel, bezogen auf die gesamten Monomeren, verzweigt bzw. vernetzt ist.

Die vorstehend genannte Emulgatorkombination bzw. der vorstehend genannte Polyelektrolyt können erfindungsgemäß zur Herstellung eines Verdickungsmittels verwendet werden.

Die vorliegende Erfindung betrifft ferner ein Verfahren zur Herstellung der erfindungsgemäßen Zusammensetzung, die zur Verwendung als Verdickungsmittel geeignet ist, bei dem der mindestens eine W/O-Emulgator und der mindestens eine Fettsäureester wie vorstehend definiert als Ölphase miteinander vermischt werden, die Monomere AMPS-Salz und Acrylsäure in teilweise neutralisierter From einschließlich Vernetzungsmittel separat in wässriger Phase miteinander vermischt werden, die wässrige Phase anschließend in der Ölphase emulgiert wird, die Monomermischung sodann polymerisiert wird und schließlich gegebenenfalls der mindestens eine O/W-Emulgator zugesetzt wird.

Schließlich betrifft die vorliegende Erfindung die Verwendung der erfindungsgemäßen Zusammensetzung als Verdickungsmittel.

Weitere bevorzugte Ausführungsformen der Erfindung sind in den abhängigen Ansprüchen beschrieben.

Unter einem inversen Latex wird im Allgemeinen eine Dispersion (Emulsion) von Polymeren in einem hydrophoben Medium (nachstehend auch als ölige Phase, Ölphase oder öliges Medium bezeichnet) verstanden, wobei die hydrophobe Phase die kontinuierliche Phase bildet.

Unter Latex wird eine Dispersion (Emulsion) von Polymeren in einem wässrigen Medium (wässriger Phase) als kontinuierlicher Phase verstanden. Siehe die entsprechende Darstellung in Römpp Chemie-Lexikon, 9. Auflage, Georg Thieme Verlag Stuttgart, New York, 1990, Seite 2459, Eintrag "Latex".

Unter einem Wasser-in-Öl-Emulgator (W/O-Emulgator) wird ein Emulgator mit einem HLB-Wert verstanden, der niedrig genug ist, um Wasser-in-Öl-Emulsionen zu ergeben. Der HLB-Wert eines solchen Emulgators ist üblicherweise kleiner als etwa 6 und liegt häufig im Bereich von 3 bis 6.

Unter einem Öl-in-Wasser-Emulgator (O/W-Emulgator) wird ein Emulgator verstanden, der einen HLB-Wert aufweist, der hoch genug ist, um Öl-in-Wasser-Emulsionen zu ergeben. Der HLB-Wert eines solchen Emulgators ist normalerweise größer als etwa 8 und liegt häufig im Bereich von 8 bis 18.

Je nach Herstellung und Zusatz der Emulgatoren kann es zu Umkehrungen der Phasenverhältnisse bei den Emulsionen kommen. Dies beschränkt jedoch nicht den Schutzbereich der vorliegenden Erfindung. Wesentlich ist, dass die erfindungsgemäße Zusammensetzung bei Einarbeitung in kosmetische Formulierungen den polymeren Bestandteil in eine kontinuierliche wäßrige Phase freisetzt, so dass er dort die gewünschte Verdickungswirkung entfalten kann.

Die erfindungsgemäße Zusammensetzung weist bezüglich ihrer Verwendung als Verdickungsmittel oder Stabilisator insbesondere die folgenden Vorteile auf:
- Sie ist schnell zu dispergieren.
- Sie kann ohne vorherige Neutralisation angewendet werden.
- Das unter Verwendung der Zusammensetzung vollständig formulierte Körperhygiene- oder pharmazeutische Produkt erfüllt alle gesetzlich festgelegten und toxikologischen Verwendungsbedingungen.
- Sie ist hochwirksam, d. h. die maximal zugegeben Menge der Zusammensetzung ist kleiner 5 Gew.-%, bezogen auf das Gesamtgewicht der Zusammensetzung, z. B. wird bei Raumtemperatur in deionisiertem Wasser bei einer Einsatzkonzentration von 2 Gew.% eine Viskosität von min. 3.000 mPas erreicht.
- Die kosmetische Formulierung, in die sie eingearbeitet worden ist, zeigt nur eine minimale Viskositätsänderung über einen breiten pH-Bereich von etwa 5 bis 11, woraus eine besonders gute Einsatzfähigkeit in Körperhygieneprodukten resultiert, da diese vorzugsweise einen pH-Bereich von 5 bis 9 aufweisen. Insbesondere weist die erfindungsgemäße Zusammensetzung bei einer Einsatzkonzentration von 2 Gew.% in deionisiertem Wasser einen pH-Wert von 5,5 bis 6,0 auf.
- Sie bleibt in kosmetischen Formulierungen wasserlöslich, so dass sie leicht von der Haut abgewaschen werden kann.
- Sie verdickt und stabilisiert neben wässrigen Lösungen auch alkoholische oder glycolische Lösungen.
- Sie ist unter den Verwendungsbedingungen UV- und temperaturbeständig. (Ein geeigneter beschleunigter Test für die Prüfung der Stabilität wäre bei höherer Temperatur für mindestens einen Monat bei 40°C in einer formulierten Zusammensetzung durchzuführen und für die tiefen Temperaturen wäre die Stabilität für mindestens drei Gefrier-Tau-Cyclen zu prüfen.)
- Sie enthält keine krebserzeugenden Rohstoffe, so dass auch im kosmetischen oder pharmazeutischen Endprodukt keine Spuren dieser Rohstoffe zurückbleiben. Insbesondere konnte das krebserzeugende Acrylamid durch Acrylsäure ersetzt werden.

Die vorstehend und in den Patentansprüchen beschriebene Erfindung betrifft also eine stabile Zusammensetzung, die zur Verwendung als Verdickungsmittel geeignet ist, und die auf einem wasserlöslichen Copolymer beruht, das in öliger Phase emulgiert ist, wobei letztere Phase aus einem Fettsäureester wie hierin definiert, am meisten bevorzugt Isopropylmyristat, besteht. Im folgenden werden allgemeine und bevorzugte Aspekte der vorliegenden Erfindung näher beschrieben.

Die erfindungsgemäße Zusammensetzung wird mittels der sogenannten inversen Emulsionspolymerisation hergestellt, einem Verfahren, das an sich im Stand der Technik bekannt ist. Bei dieser Art der Polymerisation handelt es sich um Verfahren, bei dem hydrophile Monomere in wässriger Lösung, die als diskontinuierliche Phase mit Hilfe von Wasser-in-Öl-Emulgatoren in einer mit Wasser nicht mischbaren hydrophoben Flüssigkeit als kontinuierlicher Phase emulgiert ist, polymerisiert werden. Das Reaktionsprodukt kann als inverser Latex bezeichnet werden.

Während der Polymerisation werden die Monomere der wässrigen Phase, welche in dieser im Allgemeinen in hohen Konzentrationen vorliegen, zu Teilchen mit hohen molaren Massen umgesetzt. Bei konventionellen Polymerisationen würde dies zu einem enormen Viskositätsanstieg führen, der wiederum Durchmischungsprobleme und Schwierigkeiten bei der Abführung der entstehenden Reaktionswärme nach sich ziehen würde. Konventionelle Verfahren erfordern daher den Einsatz sehr aufwendiger Polymerisationsreaktoren, wenn kleine Umsätze mit niedrigen Polymergehalten vermieden werden sollen. Die inverse Emulsionspolymerisation hat den Vorteil, dass die Reaktionsmischung niedrig viskos bleibt, auch dann, wenn die Monomeren in der wässrigen Phase hochkonzentriert zu Polymeren mit hohem Molgewicht umgesetzt werden. D. h. die Viskosität der Dispersion ist unabhängig vom Polymerisationsgrad, die Reaktionswärme kann kontrolliert abgeführt werden. Erst beim Eingießen des resultierenden Systems in Wasser invertiert die Emulsion und führt dann zu starker Verdickung, welche an dieser Stelle die gewünschte Eigenschaft des Produktes ist.

Erfindungsgemäß wird als Hauptbestandteil für die Ölphase der Polymerisation mindestens ein Fettsäureester eingesetzt, der ausgewählt ist aus der Gruppe bestehend aus den Estern von C₁-bis C₅-Alkoholen und gesättigten oder ungesättigten C₁₀- bis C₂₂-Fettsäuren, vorzugsweise C₁- bis C₃-Alkoholen mit C₁₀- bis C₂₂-Fettsäuren, insbesondere C₁₃- bis C₁₅-Fettsäuren. Insbesondere ist der Fettsäureester ausgewählt aus der Gruppe bestehend aus Estern von Methanol, Ethanol, Propanol, Isopropanol, Butanol, Butan-2-ol, tert.-Butylalkohol mit Undecansäure, Laurinsäure, Tridecansäure, Myristinsäure, Pentadecansäure oder Palmitinsäure. Dieser Bestandteil wird z. B. in einer Menge von 80 Gew.-% oder mehr, vorzugsweise 85 Gew.-% oder mehr, insbesondere 90 Gew.-% oder mehr verwendet, bezogen auf das Gewicht der Ölphase. Daneben enthält die Ölphase den mindestens einen erfindungsgemäßen W/O-Emulgator in einer Menge von im Allgemeinen bis zu 10 Gew.-%, z.B. 5 bis 10 Gew.-%, z.B. etwa 9 Gew.-%. Gegebenenfalls kann die Ölphase kleinere Mengen weiterer üblicher Zusätze enthalten.

Bei dem besonders bevorzugten Ester Isopropylmyistat handelt es sich um einen Ester der in Butter, Palmkernöl oder Kokosfett vorkommenden Myristinsäure. Isopropylmyristat ist ein natürliches und zudem umweltverträgliches Öl. Mit diesem Öl ist es gelungen, von den aus dem Stand der Technik bekannten mineralischen Ölen auf ein Naturprodukt umzusteigen, welches aus nachwachsenden Rohstoffen gewonnen wird, ohne einen Einbruch in der Qualität hinsichtlich Reproduzierbarkeit, Stabilität und Eigenschaftsprofil hinnehmen zu müssen. Denn das Isopropylmyristat ist ein Ester der z. B. in Butter, Palmkernöl und Kokosfett vorkommenden Myristinsäure mit Isopropylalkohol. Isopropylmyristat ist eine Ölkomponente, die in der Kosmetik als Spreit- und Rückfettungsmittel sowie als Lösungsvermittler eingesetzt wird. Ein Spreitmittel sorgt für eine gute Verteilung eines Öles oder Fettes auf der Haut. Es ist hautverträglich und dringt leicht in die Haut ein. Diese Eigenschaften verleihen dem hier entwickelten Produkt ein zusätzlich positives Eigenschaftsprofil für den Einsatz in kosmetischen Formulierungen.

Die für die inverse Emulsionspolymerisation geeignete wässrige Phase besteht aus 30 bis 80% Gew.-%, vorzugsweise 50 bis 70 Gew.-%, insbesondere 50 bis 55 Gew.-%, z. B. 51 Gew.-% Monomeren, bezogen auf das Gesamtgewicht der wässrigen Phase. Die erfindungsgemäße Monomermischung wird im Allgemeinen in einem Gewichtsverhältnis im Bereich von 5:1 bis 0,5:1, vorzugsweise 3:1 bis 1:1, insbesondere in einem Verhältnis von etwa 3:2 bis 5:4 aus einem 2-Acrylamido-2-methylpropansulfonsäure-Salz, z. B. einem Alkalimetallsalz oder einem Ammoniumsalz, insbesondere 2-Acrylamido-2-methylpropansulfonsäure-Natriumsalz, und Acrylsäure hergestellt, wobei die Acrylsäure auf einen pH-Wert von 3,5 bis 7, vorzugsweise 4,5 bis 6 neutralisiert wird. Zur Neutralisierung wird vorzugsweise eine Alkalilauge verwendet, insbesondere Natronlauge. Die Neutralisierung der Acrylsäure kann vor oder nach dem Mischen der Monomere erfolgen.

Zur Verzweigung bzw. Vernetzung werden ferner 0,05 bis 2 mmol, insbesondere 0,2 bis 1,5 mmol, bezogen auf die Gesamtmenge Monomeren, eines Verzweigungs- bzw. Vernetzungsmittels eingesetzt.

Dieses ist vorzugsweise Diallylharnstoff, Ethylenglycoldiacrylat, Ethylenglycolmethacrylat, N,N-Methylen-bis-acrylamid (nachstehend auch kurz "Methylenbisacrylamid"), Natriumdiallyloxyacetat, Triallylamin oder Trimethylpropantriacrylat. Die Verwendung von N,N-Methylenbisarcylamid oder Triallylamin wird besonders bevorzugt. Es können auch Kombinationen von verschiedenen der vorstehend genannten Stoffe eingesetzt werden.

Beispielhaft sei in Bezug auf das vernetzende Monomer N,N-Methylen-bis-acrylamid erläutert, dass dieses Monomer ungesättigte Stellen zum Vernetzen enthält und ausreichend wasserlöslich ist, um mit neutralisierter bzw. teilweise neutralisierter Acrylsäure und 2-Acrylamido-2-methylpropansulfonsäure bzw. deren Salzen zu reagieren. Das daraus entstehende teilweise vernetzte Copolymer ist in der Regel noch immer wasserlöslich. Zur Beibehaltung der Löslichkeit und Erzielung der optimalen Eindicker/Stabilisator-Effizienz ist das Molverhältnis des Vernetzungsmittels zum Monomergemisch von Bedeutung. Für Methylenbisacrylamid können z. B. innerhalb des Bereiches von 0,06 bis 1 Millimol Methylenbisacrylamid/Mol des Monomerengemisches gute Ergebnisse erzielt werden.

Die Reaktionsmischung kann außerdem ein oder mehrere Additive enthalten, die insbesondere unter Komplexbildnern, Kettenreglern, Initiatoren und Redoxpartnern ausgewählt werden. Weitere Komponenten, die dem Reaktionsgemisch gegebenenfalls hinzugefügt werden, sind Gelatisierungsmittel wie Ethylendiamintetraessigsäure oder Diethylentriaminpentaessigsäure.

Die Polymerisation wird in an sich bekannter Weise mit Hilfe eines freie Radikale bildenden Initiators ausgelöst, der in der wässrigen Phase und/oder in der organischen Phase gelöst sein kann, oder einer Kombination von Initiatoren. Bevorzugte Starter sind 4,4-Azobis-4-cyanovaleriansäure, 2,2'-Dimethyl-2,2'-azodi-propiononitril, 2,2-Azobis-(2,4 Diumethylvaleronitril), t-Butylhydroperoxid, Kaliumpersulfat, Cumylhydroperoxid, Benzoylperoxid sowie Redoxsysteme wie Kaliumpersulfat/Natriumbisulfit und dergleichen.

Nach ihrer Herstellung werden die wässrige Phase und die Ölphase miteinander emulgiert, so dass eine stabile Wasser-in-Öl-Emulsion erhalten wird. Dabei wird ein entsprechender Emulgator eingesetzt.

Die inverse Emulsionspolymerisation stellt besondere Ansprüche an den Emulgator, um ein stabiles System zu erhalten. Daher ist die bei bzw. nach Abschluss der Polymerisation eingesetzte Emulgatorkombination ein weiterer Aspekt der vorliegenden Erfindung. Bei den bislang üblicherweise eingesetzten Emulgatoren handelte es sich um eine Kombination aus Sorbitanmonooleat und polyethoxiliertes Sorbitanmonooleat, siehe z. B. die Offenbarung in US 6,197,287 B1.

Der erfindungsgemäße W/O-Emulgator basiert insbesondere auf einem Bernsteinsäurederivat ausgewählt aus der Gruppe bestehend aus stickstoffhaltigen Polyisobutenyl-substituierten Bernsteinsäureestern, -halbestern, -salzen, -halbsalzen, Mischformen dieser Verbindungen und Mischungen davon. Vorzugsweise weist der Polyisobutenylrest ein durchschnittliches Molekulargewicht im Bereich von 500 bis 5000, insbesondere 1000 bis 3000, wie 2000, insbesondere von 1000 (Zahlenmittel) auf. Vorzugsweise ist der Stickstoff in dem bzw. den Esterresten und/oder in den Gegenionen zu dem Salz/Halbsalz vorhanden. Insbesondere ist das Bernsteinsäurederivat ein Bernsteinsäurehalbsalz/-halbester, wobei der Esterrest oder das Gegenion zum Halbsalz abgeleitet ist von einem Amin aus der Gruppe bestehend aus mit C₁- bis C₅- Alkyl- oder Hydroxyalkylresten substituierten Aminen, insbesondere Triethylamin und Diethylaminoethanol. Am meisten bevorzugt ist der W/O-Emulgator der Stoff mit der INCI-Bezeichnung Hdroxyethyldiethonium Polyisobutenyl Triethylaminosuccinate, der in Form des Handelsprodukts Lubrizol 5603 von der Lubrizol Deutschland GmbH, Hamburg kommerziell erhältlich ist.

Der erfindungsgemäße Emulgator zeichnet sich durch seine Fähigkeit aus, hoch stabile Wasser-in-Öl-Emulsionen zu bilden die bis zu einem internen Wassergehalt von bis zu 95 Gew.-% stabil sind.

Nach abgeschlossener Polymerisation wird der inverse Latex mit dem erfindungsgemäßen Öl-in-Wasser Emulgator versetzt und ein gebrauchsfertiges Verdickungsmittel erhalten.

Der erfindungsgemäße O/W-Emulgator ist beispielsweise ein nichtionogener Emulgator mit einem HLB-Wert im Bereich von 10 bis 15, z. B. insbesondere 13, und ist insbesondere ausgewählt aus der Gruppe bestehend aus ethoxylierten primären oder sekundären C₁₀-bis C₂₀-Alkoholen, insbesondere primären oder sekundären C₁₁- bis C₁₅-Alkoholen. Sekundäre Alkohle sind bevorzugt. Vorzugsweise ist der ethoxylierte Alkohol mit durchschnittlich 1 bis 30, vorzugsweise 5 bis 20 und insbesondere etwa 9 Molen Ethylenoxid ethoxyliert. Ein besonders bevorzugter O/W-Emulgator ist als Handelsprodukt Tergitol 15-S-9 von der Firma Dow erhältlich und weist einen HLB-Wert von etwa 13 auf.

Der O/W-Emulgator kann auch der Gruppe der anionischen Tenside angehören und zwar vorzugsweise speziell aus der Gruppe der modifizierten Sulfocarbonsäureester (auch Sulfosuccinate genannt) ausgewählt sein. Insbesondere kann es sich bei dem O/W-Emulgator um ein Sulfobernsteinsäureester-Natriumsalz handeln. Das heißt, es kann sich um einen Bernsteinsäure-Voll- oder Halbester handeln, der über eine anionische Sulfonsäuregruppe mit einem geeigneten Gegenion verfügt. Zum Beispiel kann Natrium als Gegenion verwendet werden. Alternativ können andere einwertige Metallkationen oder Ammoniumionen eingesetzt werden.

Die so hergestellte erfindungsgemäße Zusammensetzung enthält im Allgemeinen
(a) 0,5 Gew.-% bis 10 Gew.-% W/O-Emulgator,
(b) 0,5 Gew.-% bis 10 Gew.-% O/W-Emulgator,
(c) 20 bis 70 Gew.-% anionischen Polyelektrolyt und
(d) mindestens 15 Gew.-% Fettsäureester.

Vorzugsweise enthält die erfindungsgemäße Zusammensetzung
(a) 1 Gew.-% bis 3 Gew.-%, insbesondere 1,5 Gew.-% bis 2 Gew.-%, insbesondere 1,8 Gew.-% W/O-Emulgator,
(b) 1 Gew.-% bis 3 Gew.-%, insbesondere 1,5 Gew.-% bis 5 Gew.-%, insbesondere 4 Gew.-% O/W-Emulgator,
(c) 30 bis 60 Gew.-%, insbesondere etwa 40 oder 41 Gew.-% anionischen Polyelektrolyt, und
(d) mindestens 20 Gew.-% Fettsäureester.

Die wie vorstehend beschrieben hergestellte erfindungsgemäße Zusammensetzung kann dann ohne weitere spezielle Vorbehandlungen in Konzentrationen von 0,2 - 5 Gew.-%, insbesondere 1 bis 3 Gew.-%, insbesondere etwa 2 Gew.-%, kosmetischen oder pharmazeutischen Formulierungen (Endprodukte) wie sogenannten Rinse-off-Produkten wie Shampoos zugesetzt werden. Die Einarbeitung in kosmetische, pharmazeutische oder andere Endprodukte, im Allgemeinen in Form von Emulsionen, kann beispielsweise auf die folgenden Arten durchgeführt werden.

Das erfindungsgemäße Verdickungsmittel kann bei einer Temperatur im Bereich von 50 bis 60°C oder auch bei einer Temperatur von etwa 40°C zu der herzustellenden Emulsion, die übliche kosmetische oder pharmazeutische Wirk- und Hilfsstoffe enthält, gegeben werden. Dabei kann die Mischung zunächst klumpen. Bei der Zugabe im Temperaturbereich 50 bis 60°C kann das erfindungsgemäße Verdickungsmittel dann sofort mittels Homogenisator eingearbeitet werden. Bei der Zugabe bei 40°C ist es bevorzugt, das erfindungsgemäße Verdickungsmittel zuerst zügig bis zum Erreichen der gewünschten Viskosität einzurühren und dann erst zu homogenisieren.

Das erfindungsgemäße Verdickungsmittel kann bei der Herstellung von Emulsionen auch vor dem Emulgieren zur Fettphase gegeben werden. Ein Homogenisieren nach der Emulsionsbildung kann dann komplett entfallen. Jedoch kann hier wie auch bei den anderen Zugabe-Varianten ein Homogenisierschritt bei Raumtemperatur ratsam sein, um die Teilchengröße zu optimieren.

Die folgenden Beispiele veranschaulichen die Erfindung.

### Beispiel 1: Herstellungsverfahren

Falls nicht ausdrücklich anders angegeben, beziehen sich die nachstehenden Mengenangaben (Teile) auf das Gewicht. In einem doppelwandigem Reaktionsgefäß, ausgestattet mit einem Rührer, einem Thermometer, einer Stickstoffspülung und zwei Dosiereinlässen wurden 91,4 Teile Isopropylmyristat vorgelegt und mit 8,6 Teilen Hydroxyethyldiethonium Polyisobutenyl Triethylaminosuccinate gemischt. Zu dieser Mischung wurden 0,1 Gew.-% 2,2'-Dimethyl-2,2'-azodipropiononitril hinzugegeben. Gemischt wurde mit einem Ultraturax T50 bei 3000 u/min bis das 2,2'-Dimethyl-2,2'-azodipropiononitril vollständig gelöst war.

In einem separaten Behälter wurden 48 Teile deionisiertes Wasser, 23 Teile Acrylsäure und 28,9 Teile 2-Acrylamido-2-methylpropansulfonsäure Natriumsalz, 0,1 Teile Diethylentriaminpentaessigsäure und 0,44 mmol Bisacrylamid, bezogen auf 1 mol der Monomere, gegeben und mit Natronlauge auf einen pH-Wert von 5,0 eingestellt.

Die wässrige Phase wurde nun langsam unter turbulentem Mischen mittels Ultraturrax T50 bei ca. 4000 U/min in das Reaktionsgefäß zu der öligen Phase hinzugegeben, wobei das Volumenverhältnis der Ölphase zur wässrigen Phase auf 30 : 70 eingestellt wurde. Anschließend wurde noch ca. 5 min. bei ca. 6000 U/min emulgiert, bis eine stabile, cremige W/O-Emulsion entstand. Das Reaktionsgefäß wurde verschlossen und bis zur vollständigen Entfernung des Luftsauerstoffes mit Stickstoff durchspült. Anschließend wurde das Reaktionsgemisch auf eine Temperatur von 30°C gebracht und die Polymerisation wurde durch weitere Zugabe von Cumolhydroperoxid als Radikalstarter mit Natriumdisulfit als Redoxpartner gestartet. Es kam zu einer spontanen stark exothermen Reaktion. Zur Vervollständigung der Reaktion und Reduzierung des Restmonomergehaltes auf ein Minimum wurde nach Erhöhung der Temperatur auf 70°C erneut eine Portion des Radikalstarters und des Redoxpartners hinzugegeben. Nach beendeter Reaktion wurde das Reaktionsgemisch auf eine Temperatur von 25°C heruntergekühlt und 4 Teile des nichtionischen O/W-Emulgators Tergitol^{™} 15-S-9 wurden unter starkem Rühren hinzugefügt. Das so erhaltene Produkt konnte ohne weitere Behandlung zu Körperhygiene- oder pharmazeutischen Produkten zugefügt werden.

### Beispiele 2 bis 10

Im Folgenden werden Beispiele für Formulierungen angegeben, die unter Verwendung der erfindungsgemäßen Zusammensetzung hergestellt werden können:

Die Viskositäten und pH-Werte der Formulierungen sind nach 3-monatiger Lagerzeit stabil.

### Beispiel 2

| **Körperlotion** | | | | |
|---|---|---|---|---|
| **Phase** | **Rohstoff** | **INCI** | **Lieferant** | **%** |
| A | Deionised Water | Aqua | | 78,00 |
| | Euxyl PE 9010 | Phenoxyethanol 90%, Ethylhexyl-glycerin 10% | Schülke & Mayr | 1,00 |
| | | | | |
| B | Tego Care 450 (Polyglyce-rin-Zuckerester, nichtion. HLB 12) | Polyglyceryl-3 Methylglucose Distearate | Degussa | 2,00 |
| | Myritol 318/ Neutralöl pflanzl. | Caprylic/Capric Triglyceride | Cognis/ Symrise | 8,00 |
| | Squalan Pflanzl. Ph.Eur.4.04 | Squalane, veg.grade | Worlée | 2,00 |
| | Sonnenblumenöl raff. | Helianthus Annuus | | 6,00 |
| | Cetiol OE | Dicaprylyl Ether | Cognis | 2,00 |
| | | | | |
| C | **WorleeGel COS 1411** | Sodium Acrylate /Sodium Acryloyl-dimethyl Taurate Copolymer (42%AS), Isopropyl Myristate, Hydroxyethyldiethoniumpolyisobutenyl-triethylaminosuccinate, C11-15 Pareth-9 (d.h. Tergitol 15-S-9) | Worlée | **1,00** |
| | | | | **100,00** |

| **Spezifikation:** | |
|---|---|
| Aussehen: | weiße, leicht durchscheinende, zähfließende Emulsion |
| pH-Wert n.d. Herstellung: Optimum pH 6,5 - 7 | 6,4 |
| pH-Wert n.3 Mon RT: | 6,13 |
| Viskosität (Brookfield: Spindle 6; Speed 10):mpas n.d. Herstellung | 14200 |
| Viskosität (Brookfield: Spindle 6; Speed 10):mpas n.3 Mon. RT | 18000 |
| Zentrifugentest (5000upm, 2900RZB, 10min.): | ok |
| Mikroskop (630x): | Feinverteilung sehr gut |
| **Stabilität:** | |
| 20°C: | > 3 Mon.ok |
| 40°C: | >3 Mon ok |
| 4°C: | > 3 Mon.ok |
| Wechseltemperaturbelastung (Schaukeltest): | > 5 x ok |

### Herstellvorschrift:

Die Bestandteile der Phase A wurden auf 78°C erwärmt. Die Phase B wurde auf 75°C erwärmt. Die Phase A wurde vorgelegt, die Phase B wurde vorsichtig aufgeschichtet, dann homogenisiert. Unter Rühren wurde auf 55-40°C abgekühlt. Der Part C wurde zugegeben. Unter Rühren wurde auf 35°C gekühlt. Dann wurde homogenisiert.

Unter Rühren wurde auf RT abgekühlt. Das pH-Optimum lag bei 6,8 - 7,3.

### Beispiel 3

| **Augenpflegecreme** | | | | |
|---|---|---|---|---|
| **Phase** | **Rohstoff** | **INCI** | **Lieferant** | **%** |
| A | Deionised Water | Aqua | | 76,00 |
| | Euxyl PE 9010 | Phenoxyethanol 90%, Ethylhexylglycerin 10% | Schülke&Mayr | 1,00 |
| A1 | Sisterna SP 70 C | Sucrose Stearate | Sisterna | 1,00 |
| | Sisterna SP 30 C | Sucrose Distearate | Sisterna | 3,00 |
| | | | | |
| B | Myritol 318/ Neutralöl pflanzl. | Caprylic/Capric Triglyceride | Cognis/ Symrise | 8,00 |
| | Squalan Pflanzl. Ph.Eur.4.04 | Squalane, veg.grade | Worlée | 2,00 |
| | Sonnenblumenöl raff. | Helianthus Annuus | | 6,00 |
| | Cetiol OE | Dicaprylyl Ether | Cognis | 2,00 |
| | | | | |
| C | **WorleeGel COS 1411** | Sodium Acrylate /Sodium Acryloyldimethyl Taurate Copolymer (42%AS), Isopropyl Myristate, Hydroxyethyldiethoniumpolyiso-butenyltriethylaminosuccinate, C11-15 Pareth-9 | Worlée | **1,00** |
| | | | | **100,00** |

| **Spezifikation:** | |
|---|---|
| Aussehen: | weiße, lufthaltige, weiche Emulsion, nicht fließfähig, leicht unruhig |
| pH-Wert n.d. Herstellung: Optimum pH 6,5 - 7 | 6,3 |
| pH-Wert n.3 Mon RT: | 6,3 |
| pH-Wert n.3 Mon 40°: | 6,2 |
| Viskosität (Brookfield: Spindle 6; Speed 10):mpas n.d. Herstellung | 22700 |
| Viskosität (Brookfield: Spindle 6; Speed 10):mpas n.3 Mon. RT | 22000 |
| Zentrifugentest (5000upm, 2900RZB, 10min.): | ok |
| Mikroskop (630x): | Feinverteilung sehr gut |
| **Stabilität:** | |
| 20°C: | n.3 Mon.ok |
| 40°C: | vereinzelt Agglomerate nach 3 Monaten |
| 4°C: | n.3 Mon.ok |
| Wechseltemperaturbelastung (Schaukeltest): | > 3x ok |

### Herstellvorschrift:

Die Bestandteile der Phase A wurden auf 75°C erwärmt. Dann wurde kurz homogenisiert. Die Phase B wurde auf 72°C erwärmt. Die Phase A wurde vorgelegt, die Phase B unter Rühren zugegeben, dann ausreichend homogenisiert. Unter Rühren wurde auf 55-40°C abgekühlt. Der Part C wurde unter Rühren zugegeben und ca. 5-10 Min. weitergerührt. Bei ca. 35° wurde ausreichend homogenisiert. Dann wurde unter Rühren auf RT abgekühlt. Es empfiehlt sich, den pH-Wert auf 6,5 - 6,8.

### Abweichende Herstellungsmöglichkeiten

Zugabe Phase C in die heiße Ölphase incl. Sisterna SP 30 (sonst Verklumpung).

### Beispiel 4

| **Körperbalsam** | | | | |
|---|---|---|---|---|
| **Phase** | **Rohstoff** | **INCI** | **Lieferant** | **%** |
| A | Deionised Water | Aqua | | 76,00 |
| | Euxyl PE 9010 | Phenoxyethanol 90%, Ethylhexylglycerin 10% | Schülke&Mayr | 1,00 |
| | | | | |
| B | Amphisol K (Phosphatester, anion.) | Potassium Cetyl Phosphate | Uniqema | 2,00 |
| | Myritol 318/ Neutralöl pflanzl. | Caprylic/Capric Triglyceride | Cognis/ Symrise | 8,00 |
| | Squalan Pflanzl. Ph.Eur.4.04 | Squalane, veg.grade | Worlée | 2,00 |
| | Sonnenblumenöl raff. | Helianthus Annuus | | 6,00 |
| | Cetiol OE | Dicaprylyl Ether | Cognis | 2,00 |
| | | | | |
| C | **WorleeGel COS 1411** | Sodium Acrylate /Sodium Acryloyldimethyl Taurate Copolymer (42%AS), Isopropyl Myristate, Hydroxyethyldiethoniumpolyisobutenyltriethylaminosuccinate, C11-15 Pareth-9 | Worlée | **3,00** |
| | | | | **100,00** |

| **Spezifikation:** | | | | |
|---|---|---|---|---|
| Aussehen: | | | | lufthaltige, viskose Emulsion |
| pH-Wert n.d. Herstellung: | | | | 6,5 |
| pH-Wert n.3 Mon RT: | | | | 6,67 |
| Viskosität (Brookfield: Spindle 6; Speed 10): mpas n. d. Herstellung | | | | 22500 |
| Viskosität (Brookfield: Spindle 6; Speed 10): mpas n. 3 Mon. RT | | | | 22500 |
| Zentrifugentest (5000upm, 2900RZB, 10 min.): | | | | ok |
| Mikroskop (630x): | | | | Feinverteilung ok |

| **Stabilität:** | | | | |
|---|---|---|---|---|
| 20°C: | | | | > 3 Mon.ok |
| 40°C: | | | | > 3 Mon.ok |
| 4°C: | | | | > 3 Mon.ok |
| Wechseltemperaturbelastung (Schaukeltest): | | | | etwas Öl an Oberfläche n. 8 Tagen |

### Herstellvorschrift:

Die Bestandteile der Phase A wurden auf 80°C erwärmt. Die Phase B wurde auf 75°C erwärmt, kurz vor dem Zusammengeben wurde ca. 5 % von der Phase A zu B gegeben und gut gemischt. Die Phase B wurde vorgelegt, die Phase A unter Rühren zugegeben und ausreichend homogenisiert. Unter Rühren wurde auf 55-40°C abgekühlt, der Part C zugegeben. Unter Rühren wurde weitergekühlt auf 35 °C, dann homogenisiert. Unter Rühren wurde auf RT abgekühlt.

### Abweichende Herstellungsmöglichkeiten

Zugabe der Phase C in die heiße Ölphase. Dann bei RT nachhomogenisieren, um die Teilchengröße zu optimieren.

### Beispiel 5

| **Soft Cream** | | | | |
|---|---|---|---|---|
| **Phase** | **Rohstoff** | **INCI** | **Lieferant** | **%** |
| A | Deionised Water | Aqua | | 74,75 |
| | Euxyl PE 9010 | Phenoxyethanol 90%, Ethylhexylglycerin 10% | Schülke & Mayr | 1,00 |
| | | | | |
| B | Montanov L (Zuckerether, nichtionisch, HLB | C14-22 Alcohols, C12-20 Alkylglucoside (100%) | Seppic | 2,00 |
| | Myritol 318/ Neutralöl pflanzl. | Caprylic/Capric Triglyceride | Cognis/ Symrise | 8,00 |
| | Squalan Pflanzl. Ph.Eur.4.04 | Squalane, veg.grade | Worlèe | 3,00 |
| | Sonnenblumenöl raff. | Helianthus Annuus | | 5,00 |
| | Cetiol OE | Dicaprylyl Ether | Cognis | 2,00 |
| | | | | |
| C | **WorleeGel COS 1411** | Sodium Acrylate /Sodium Acryloyldimethyl Taurate Copolymer (42%AS), Isopropyl Myristate, Hydroxyethyldiethoniumpolyisobutenyltriethylaminosuccinate, C11-15 Pareth-9 | Worlée | **4,00** |
| D | Sodium Hydroxide (sol.10%) | Sodium Hydroxide | Merck | 0,25 |
| | | | | **100,00** |

| **Spezifikation:** | |
|---|---|
| Aussehen: | weiße, viskose Emulsion |
| pH-Wert n.d. Herstellung: | 5,95 (ohne NaOH pH 5,7) |
| pH-Wert n.3 Mon RT: | 6,2 |
| Viskosität (Brookfield: Spindle 6; Speed 10):mpas n.d. Herstellung | 31700 |
| Viskosität (Brookfield: Spindle 6; Speed 10):mpas n.3 Mon. RT | 25400 |
| Zentrifugentest (5000upm, 2900RZB, 10min.): | ok |
| Mikroskop (630x): | Feinverteilung ok |

| **Stabilität:** | |
|---|---|
| 20°C: | >3 Mon ok |
| 40°C: | >3 Mon ok |
| 4°C: | >3 Mon ok |
| Wechseltemperaturbelastung (Schaukeltest): | > 3 Zyklen ok |

### Herstellvorschrift:

Die Bestandteile der Phase A wurden auf 75°C erwärmt. Die Phase B wurde auf 72°C erwärmt. Die Phase A wurde vorgelegt, Phase B unter Rühren zugegeben. Dann wurde ausreichend homogenisiert. Unter Rühren wurde auf 50°C abgekühlt. Dann wurde nochmals kurz homogenisiert. Der Part C wurde zugegeben und unter Rühren vollständig gelöst. Dabei wurde weitergekühlt auf Raumtemperatur.

### Beispiel 6

| **Pflegecreme** | | | | |
|---|---|---|---|---|
| **Phase** | **Rohstoff** | **INCI** | **Lieferant** | **%** |
| A | Deionised Water | | | 76,00 |
| | Euxyl PE 9010 | Phenoxyethanol 90%, Ethylhexylglycerin 10% | Schülke & Mayr | 1,00 |
| | | | | |
| B | Dermofeel PS (Polyglycerinester, nichtion., HLB 9) | Polyglyceryl-3 Stearate | Dr.Straetmans | 2,00 |
| | Myritol 318/ Neutralöl pflanzl. | Caprylic/Capric Triglyceride | Cognis/ Symrise | 8,00 |
| | Squalan Pflanzl. Ph.Eur.4.04 | Squalane, veg.grade | Worlée | 2,00 |
| | Sonnenblumenöl raff. | Helianthus Annuus | | 6,00 |
| | Cetiol OE | Dicaprylyl Ether | Cognis | 2,00 |
| | | | | |
| C | **WorleeGel COS 1411** | Sodium Acrylate /Sodium Acryloyldimethyl Taurate Copolymer (42%AS), Isopropyl Myristate, Hydroxyethyldiethoniumpolyisobutenyltriethylaminosuccinate, C 11-15 Pareth-9 | Worlée | **3,00** |
| | | | | **100,00** |

| **Spezifikation** | |
|---|---|
| Aussehen: | weiße, glatte, viskose Emulsion |
| pH-Wert nach der Herstellung: | 7,3 |
| pH-Wert nach 3 Mon. RT: | 7,39 |
| Viskosität (Brookfield: Spindle 6; Speed 10):mpas n. d. Herstellung | 30500/28000 mpas wenn C bei 40° zugegeben wird |
| Viskosität (Brookfield: Spindle 6; Speed 10):mpas n. 3 Mon. RT | 30000/ 31000 |
| Zentrifugentest (5000upm, 2900RZB, 10min.): | ok |
| Mikroskop (630x): small droplet distribution | ok |
| | |

| **Stabilität:** | |
|---|---|
| 20°C | > 3 Mon.ok |
| 40°C | > 3 Mon.ok |
| 4°C | > 3 Mon.ok |
| Wechseltemperaturbelastung (Schaukeltest) | > 4 Zyklen ok |

### Herstellvorschrift:

Die Bestandteile der Phase A wurden auf 75°C erwärmt. Die Phase B wurde bei 72°C aufgeschmolzen. Die Phase A wurde vorgelegt, die Phase B unter Rühren zugegeben, dann wurde 1 Min. 20 Sek. homogenisiert (UT 11.000 UPM). Es wurde bis 40°C gekühlt, dann der Part C zugegeben. Unter Rühren wurde weitergekühlt auf 35°C, dann wurde homogenisiert. Der pH-Wert mit der Phase D wurde auf ca. pH 6,5 eingestellt, dann wurde 30 Sek. homogenisiert. Unter Rühren wurde auf RT abgekühlt.

### Beispiel 7

| **Protection Cream** | | | | |
|---|---|---|---|---|
| **Phase** | **Rohstoff** | **INCI** | **Lieferant** | **%** |
| A | Deionised Water | | | 75,00 |
| | Euxyl PE 9010 | Phenoxyethanol 90%, Ethylhexylglycerin 10% | Schülke & Mayr | 1,00 |
| | | | | |
| B | Dermofeel SL (Fettsäureester, anion. HLB 17) | Sodium Stearoyl Lactylate | Dr.Straetmans | 2,00 |
| | Myritol 318/ Neutralöl pflanzl. | Caprylic/Capric Triglyceride | Cognis/ Symrise | 8,00 |
| | Squalan Pflanzl. Ph.Eur.4.04 | Squalane, veg.grade | Worlée | 2,00 |
| | Sonnenblumenöl raff. | Helianthus Annuus | | 6,00 |
| | Cetiol OE | Dicaprylyl Ether | Cognis | 2,00 |
| | | | | |
| C | **WorleeGel COS 1411** | Sodium Acrylate /Sodium Acryloyldimethyl Taurate Copolymer (42%AS), Isopropyl Myristate, Hydroxyethyldiethoniumpolyisobutenyl-triethylaminosuccinate, C11-15 Pareth-9 | Worlée | **4,00** |
| | | | | **100,00** |

| **Spezifikation** | |
|---|---|
| Aussehen: | weiße, viskose Emulsion |
| pH-Wert nach der Herstellung: | 5,9 |
| pH-Wert nach 3 Mon. RT: | 6,14 |
| pH-Wert nach 6 Mon. RT: | 5,7 |
| pH-Wert nach 3 Mon. 40°C: | 5,7 |
| Viskosität (Brookfield: Spindle 6; Speed 10):mpas n.d. Herstellung | 37000 |
| Viskosität (Brookfield: Spindle 6; Speed 10):mpas 3 Monaten RT3200 | 0 |
| Zentrifugentest (5000upm, 2900RZB, 10min.): | ok |
| Mikroskop (630x): small droplet distribution i.O. | ok |

| **Stabilität:** | |
|---|---|
| 20°C | > 3 Mon.ok, pH 5,7 |
| 40°C | > 3 Mon.ok, pH 5,7 |
| 4°C | > 3 Mon.ok |
| Wechseltemperaturbelastung (Schaukeltest) | > 5 Zyklen ok |

### Herstellvorschrift:

Die Bestandteile der Phase A wurden auf 75°C erwärmt. Die Phase B wurde bei 72°C aufgeschmolzen. Die Phase A wurde vorgelegt, dann die Phase B unter Rühren zugegeben, anschließend wurde ausreichend homogenisiert. Es wurde bis bis 55-40°C gekühlt, der Part C zugegeben und unter Rühren auf 35°C abgekühlt. Dann wurde homogenisiert und abgekühlt auf RT. Unter Rühren wurde auf RT abgekühlt.

### Abweichende Herstellung

Zugabe von Phase C in die heiße Ölphase incl. Emulgator; Herstellung komplett ohne Homogenisierschritte möglich.

### Beispiel 8

| **Sun-Emulgel mit Worlée Gel** | | | | |
|---|---|---|---|---|
| **Phase** | **Ingredient** | **INCI** | **Lieferant** | **%** |
| A | Deionised Water | Aqua | | 77,50 |
| | | | | |
| B | Neo Heliopan Typ 303 | Octocrylene | Symrise6,00 | |
| | Eusolex 9020 | Butyl Methoxydibenzoylmethane | Merck | 2,00 |
| | Neo Heliopan AV | Ethylhexyl Methoxycinnamate , Octinoxate | Symrise4,00 | |
| | Tegosoft DEC | Diethylhexyl Carbonate | Degussa | 5,00 |
| | Aprikosenkernöl | Prunus Armeniaca Kernel Oil | | 1,00 |
| B1 | Pemulen TR-1 | Acrylates/C10-30 Alkyl Acrylate Crosspolymer | Gattefosse/ Noveon | 0,20 |
| | | | | |
| C | Sodium Hydroxide (sol.10%) | Sodium Hydroxide | Merck | 0,50 |
| | Euxyl PE 9010 | Phenoxyethanol 90%, Ethylhexylglycerin 10% | Schülke&Mayr | 1,00 |
| | | | | |
| D | **WorleeGel COS 1411** | Sodium Acrylate/Sodium Acryloyldimethyl Taurate Copolymer (42%AS), Isopropyl Myristate, Hydroxyethyldiethoniumpolyisobutenyltriethy-laminosuccinate, C11-15 Pareth-9 | Worlée | 1,00 |
| | | | | |
| E | Panthenol 50P | Panthenol, Propylene Glycol | Roche | 1,00 |
| | Parf. Rock & Roll 4035 | Parfum | Anessence | 0,20 |
| | Parf. Rose Water | Parfum | Special Cosmetics Ploch | |
| | | | | |
| F | Deionised Water | Aqua | | 0,50 |
| | Cranberry Spray-dried | | Worlée | 0,10 |
| | | | | **100,00** |

| **Spezifikation** | |
|---|---|
| | |
| pH Wert: | 6,1 |
| Viskosität (Brookfield spindle 6, speed 10): ca. 15000 mpas | 13900 |
| Aussehen: Weiße Emulsion mit Gelcharakter | |
| Zentrifugentest (5000 RPM; 2900 RZB; 10 min.): ok | ok |
| Stabilität: 20° | > 3 Mon.ok |
| 40° | > 3 Mon.ok, pH 5,61 |
| 4° | > 3 Mon.ok |

**Herstellung:** Das Wasser der Phase A wurde auf 70°C erwärmt. Die Phase B im Mischer wurde vorgelegt und auf 78°C erwärmt. B1 wurde kurz vor dem Emulgieren in B dispergiert. A wurde zu B unter Rühren emulgiert. Es wurde ca. 5-10 min gerührt. Dann wurde ausreichend homogenisiert. Anschließend wurde abgekühlt auf 55°C. Zuerst wurde der Part C, dann das Worlée Gel unter Rühren zugegeben. Es wurde weitergekühlt auf 40°C und die Bestandteile von E und F zugegeben. Dann wurde abgekühlt auf Raumtemperatur.

### Beispiel 9

| **Emulsion mit Worlée Nano-Emulsion Squalan** | | | | |
|---|---|---|---|---|
| **Phase** | **Ingredient** | **INCI** | **Lieferant** | **%** |
| A | Deionised Water | Aqua | Worlée | 74,20 |
| | Euxyl PE 9010 | Phenoxyethanol 90%, Ethylhexylglycerin 10% | Schülke&Mayr | 1,00 |
| | | | | |
| B | Tego Care 450 | Polyglyceryl-3 Methylglucose Distearate | Degussa | 2,00 |
| | Myritol 318/ Neutralöl pflanzl. | Caprylic/Capric Triglyceride | Cognis/ Symrise | 8,00 |
| | Cetiol OE | Dicaprylyl Ether | Cognis | 2,00 |
| | Cutina GMS | Glyceryl Stearate | Cognis | 1,00 |
| | DC 200 Fluid (100 cs) | Dimethicone | Dow Corning | 0,30 |
| | | | | |
| C | **WorleeGel COS 1411** | Sodium Acrylate /Sodium Acryloyldimethyl Taurate Copolymer (42%AS), Isopropyl Myristate, Hydroxyethyldiethonium-polyisobutenyltriethylaminosuccinate, C11-15 Pareth-9 | Worlée | 1,50 |
| | | | | |
| D | WorléeNano-Emulsion Avocado | 458000-11921, 24.7.06: Squalane, Lauryl Glucoside, Glycerin, Phenoxyethanol, Lactic Acid (Ölphase > 50%) | | 10,00 |
| | | | | **100,00** |

### Herstellung:

Die Phase A wurde auf 75°C erwärmt. Die Phase B wurde auf 75°C aufgeschmolzen. Die Phase B wurde auf die Phase A aufgeschichtet ohne Rühren. Dann wurde ausreichend homogenisiert. Es wurde auf 55°C gekühlt, dann der Part C zugegeben unter Rühren. Anschließend wurde weitergekühlt auf < 40°C und der Part D zugegeben. Dann fand eine Abschlusshomogenisation und Kühlung auf Raumtemperatur statt.

| | |
|---|---|
| pH Wert: | 6,36 |
| Viskosität (Brookfield spindle 6, speed 10): ca. 15000 mpas | 13500 |
| Aussehen: Weiße Emulsion mit Gelcharakter | |
| Zentrifugentest (5000 RPM; 2900 RZB; 10 min.): | ok |
| Stabilität: 20° | >3 Mon ok |
| 40° | >3 Mon ok |
| Wechseltemperatur/4° | >3 Mon ok |

### Beispiel 10

| **Emulgel mit Worlée Nano-Emulsion Avocado** | | | | |
|---|---|---|---|---|
| **Phase** | **Ingredient** | **INCI** | **Lieferant** | **%** |
| | Worlée Nano-Emulsion Avocado | 458000-11917, 24.7.06: Avocadoöl, Lauryl Glucoside, Glycerin, Phenoxyethanol, Lactic Acid (Ölphase > 50%) | | 10,00 |
| | Deionised Water | Aqua | Worlée | 85,50 |
| | Euxyl PE 9010 | Phenoxyethanol 90%, Ethylhexyl-glycerin 10% | Schülke & Mayr | 1,00 |
| | DC 200 Fluid (100 cs) | Dimethicone | Dow Corning | 0,50 |
| | **WorleeGel COS 1411** | Sodium Acrylate /Sodium Acry-loyldimethyl Taurate Copolymer (42%AS), Isopropyl Myristate, Hydroxyethyldiethoniumpolyiso-butenyltriethylaminosuccinate, C11-15 Pareth-9 | Worlée | 3,00 |
| | | | | |
| | | | | **100,00** |

**Herstellung:** Die Bestandteile wurden in vorgegebener Reihenfolge gemischt und bis zum glatten Gel weitergerührt.

| **Spezifikation** | |
|---|---|
| | |
| pH Wert: | 6,09 |
| Viskosität (Brookfield spindle 6, speed 10): ca. 15000 mpas | 13500 |
| Aussehen: Weiße Emulsion mit Gelcharakter | |
| Zentrifugentest (5000 RPM; 2900 RZB; 10 min.): ok | ok |
| Stabilität: 20° | >3 Mon ok |
| 40° | >3 Mon ok |
| Wechseltemperatur/4° | >3 Mon ok |

### Beispiel 11

| **Fluid mit Worlée Gel** | | | | |
|---|---|---|---|---|
| **Phase** | **Ingredient** | **INCI** | **Lieferant** | **%** |
| A | Deionised Water | Aqua | | 91,75 |
| | **WorleeGel COS 1411** | Sodium Acrylate /Sodium Acryloyldimethyl Taurate Copolymer (42%AS), Isopropyl Myristate, Hydroxyethyldiethoniumpolyiso-butenyltriethylaminosuccinate, C11-15 Pareth-9 | Worlée | 1,50 |
| | | | | |
| B | Frescolat ML | Menthyl Lactate | Symrise | 0,30 |
| | Parf. Granatapfel 66025B | Parfum | HGH Fragr.Res. | 0,15 |
| | Panthenol 50P | Panthenol, Propylene Glycol | Roche | 1,00 |
| | | | | |
| C | Twincide G | 50% Glycerin, 25% Benzyl PCA, 25% Phenoxyethanol | IMPAG/Roda | 3,00 |
| | DC 1501 Fluid | Cyclopentasiloxane, Dimethiconol | Dow Corning | 1,00 |
| | | | | |
| D | Deionised Water | Aqua | | 1,00 |
| | Cranberry Spray-dried | | Worlée | 0,30 |
| | | | | |
| E | Sodium Hydroxide (sol.10%) | Sodium Hydroxide | Merck | 0,20 |
| | | | | |
| | | | | **100,00** |

**Herstellung:** Das Wasser der Phase A wurde vorgelegt und auf 30°C erwärmt. Das Worlée Gel wurde unter Rühren zugegeben und es wurde bis zur vollständigen Quellung weitergerührt. Die Phase B wurde vorgelöst und zugegeben. Die Bestandteile von C wurden nacheinander unter Rühren zugegeben. Dann wurde abgekühlt auf Raumtemperatur und die Vorlösung D zugegeben. Der pH-Wert wurde auf ca. 6 eingestellt.

| | | | | |
|---|---|---|---|---|
| pH Wert: | | | | 5,9 |
| Viskosität (Brookfield spindle 6, speed 10): ca. 15000 mpas | | | | 2300 |
| Aussehen: Weiße Emulsion mit Gelcharakter | | | | |
| Zentrifugentest (5000 RPM; 2900 RZB; 10 min.): | | | | ok |
| Stabilität: 20° | | | | > 2 Mon.ok |
| 40° | | | | > 2 Mon.ok |
| Wechseltemperatur/4° | | | | leichte Ölabscheidung nach 3 Zyklen |

## Patentansprüche

1. Zusammensetzung enthaltend eine Ölphase, eine wässrige Phase und mindestens einen Wasser-in-Öl-Emulgator (W/O-Emulgator), wobei die wässrige Phase mindestens einen verzweigten oder vernetzten anionischen Polyelektrolyten enthält, der durch Copolymerisation von 2-Acrylamido-2-methylpropansulfonsäuresalz (AMPS-Salz) mit teilweise neutralisierter Acrylsäure hestellbar ist, und die Ölphase aus mindestens einem. Ester eines C₁- bis C₅-Alkohols und einer gesättigten oder ungesättigten C₁₀- bis C₂₂-Fettsäure besteht.

2. Zusammensetzung nach Anspruch 1, **dadurch gekennzeichnet, dass** sie ferner mindestens einen Öl-in-Wasser-Emulgator (O/W-Emulgator) enthält.

3. Zusammensetzung nach Anspruch 1 oder Anspruch 2, **dadurch gekennzeichnet, dass** das 2-Acrylamido-2-methylpropansulfonsäuresalz das Natriumsalz ist.

4. Zusammensetzung nach einem der vorhergehenden Ansprüche, **dadurch gekennzeichnet, dass** die Acrylsäure mit Natronlauge neutralisiert ist.

5. Zusammensetzung nach einem der vorhergehenden Ansprüche, **dadurch gekennzeichnet, dass** der mindestens eine W/O-Emulgator ein Bernsteinsäurederivat ausgewählt aus der Gruppe bestehend aus stickstoffhaltigen Polyisobutenyl-substituierten Bernsteinsäureestern, -halbestern, -salzen, -halbsalzen, Mischformen dieser Verbindungen und Mischungen davon ist.

6. Zusammensetzung nach Anspruch 5, **dadurch gekennzeichnet, dass** der Polyisobutenylrest ein durchschnittliches Molekulargewicht von 1000 (Zahlenmittel) aufweist.

7. Zusammensetzung nach Anspruch 5 oder Anspruch 6, **dadurch gekennzeichnet, dass** der Stickstoff in dem bzw. den Esterresten und/oder in den Gegenionen zu dem Salz/Halbsalz vorhanden ist.

8. Zusammensetzung nach Anspruch 5 oder Anspruch 6, **dadurch gekennzeichnet, dass** das Bernsteinsäurederivat ein Bernsteinsäurehalbsalz/-halbester ist, wobei der Esterrest oder das Gegenion zum Halbsalz abgeleitet ist aus der Gruppe bestehend aus Triethylamin und Dieethylaminoethanol.

9. Zusammensetzung nach Anspruch 5, **dadurch gekennzeichnet, dass** der W/O-Emulgator der Stoff mit der INCI-Bezeichnung Hydroxyethyldiethonium Polyisobutenyl Triethylaminosuccinate ist.

10. Zusammensetzung nach einem der vorhergehenden Ansprüche 2 bis 9, **dadurch gekennzeichnet, dass** der mindestens eine O/W-Emulgator ausgewählt ist aus der Gruppe bestehend aus ethoxylierten primären oder sekundären C₁₀- bis C₂₀-Alkoholen, insbesondere primären oder sekundären C₁₁- bis C₁₅-Alkoholen, anionischen Sulfosuccinaten und Mischungen davon.

11. Zusammensetzung nach Anspruch 10, **dadurch gekennzeichnet, dass** der ethoxylierte Alkohol mit durchschnittlich 1 bis 30, vorzugsweise 5 bis 20 und insbesondere etwa 9 Molen Ethylenoxid ethoxyliert ist.

12. Zusammensetzung nach einem der vorhergehenden Ansprüche, **dadurch gekennzeichnet, dass** der mindestens eine Fettsäureester der Ölphase ausgewählt ist aus der Gruppe bestehend aus den Estern von C₁- bis C₃-Alkoholen mit gesättigten C₁₀- bis C₂₂-Fettsäuren.

13. Zusammensetzung nach einem der Ansprüche 1 bis 11, **dadurch gekennzeichnet, dass** der mindestens eine Fettsäureester ausgewählt ist aus der Gruppe bestehend aus Estern von Methanol, Ethanol, Propanol, Isopropanol, Butanol, Butan-2-ol, tert.-Butylalkohol mit Undecansäure, Laurinsäure, Tridecansäure, Myristinsäure, Pentadecansäure oder Palmitinsäure.

14. Zusammensetzung nach Anspruch 13, **dadurch gekennzeichnet, dass** der Fettsäureester Isopropylmyristat ist.

15. Zusammensetzung nach einem der vorhergehenden Ansprüche 2 bis 14, **dadurch gekennzeichnet, dass** sie
(a) 0,5 Gew.-% bis 10 Gew.-% W/O-Emulgator,
(b) 0,5 Gew.-% bis 10 Gew.-% O/W-Emulgator,
(e) 20 bis 70 Gew.-% anionischen Polyelektrolyt und
(f) mindestens 15 Gew.-% Fettsäureester enthält.

16. Verfahren zur Herstellung der Zusammensetzung nach einem der Ansprüche 1 bis 15, bei dem der mindestens eine W/O-Emulgator und der mindestens eine Fettsäureester als Ölphase miteinander vermischt werden, die Monomere AMPS-Salz und Acrylsäure in teilweise neutralisierter Form einschließlich Vernetzungsmittel separat in wässriger Phase miteinander vermischt werden, die wässrige Phase anschließend in der Ölphase emulgiert wird, die Monomermischung sodann polymerisiert wird und schließlich gegebenenfalls der mindestens eine O/W-Emulgator zugesetzt wird.

17. Verwendung der Zusammensetzung gemäß den Ansprüchen 1 bis 15 oder hergestellt gemäß Anspruch 16 als Verdickungsmittel, insbesondere als Verdickungsmittel für kosmetische oder pharmazeutische Produkte, vorzugsweise ein Rinse-off-Produkt wie ein Shampoo.

## Claims

1. A composition comprising an oil phase, an aqueous phase and at least one water-in-oil emulsifier (W/O emulsifier) wherein the aqueous phase comprises at least one branched or crosslinked anionic polyelectrolyte that can be manufactured by copolymerising a salt of 2-acrylamido-2-methylpropane sulfonic acid (AMPS salt) with partially neutralised acrylic acid, and the oil phase consists of at least one ester of a C₁-C₅ alcohol and a saturated or unsaturated C₁₀-C₂₂ fatty acid.

2. The composition according to Claim 1 further comprising at least one oil-in-water emulsifier (O/W emulsifier).

3. The composition according to Claim 1 or Claim 2 **characterised in that** the salt of 2-acrylamido-2-methylpropane sulfonic acid is the sodium salt.

4. The composition according to one of the preceding claims **characterised in that** the acrylic acid is neutralised with caustic soda.

5. The composition according to one of the preceding claims **characterised in that** the at least one W/O emulsifier is a succinic acid derivative selected from the group consisting of esters, half esters, salts, half salts of nitrogen-containing polyisobutenyl-substituted succinic acids, mixed forms of these compounds and mixtures thereof.

6. The composition according to Claim 5 **characterised in that** the average molecular weight of the polyisobutenyl moiety is 1000 (number average).

7. The composition according to Claim 5 or Claim 6 **characterised in that** the nitrogen is present in the ester moiety or moieties and/or in the counter ions to the salt/half salt.

8. The composition according to Claim 5 or Claim 6 **characterised in that** the succinic acid derivative is a half salt/half ester of succinic acid, wherein the ester moiety or the counter ion to the half salt is derived from the group consisting of triethylamine and diethylaminoethanol.

9. The composition according to Claim 5 **characterised in that** the W/O emulsifier is the substance with the INCl name Hydroxyethyldiethonium Polyisobutenyl Triethylaminosuccinate.

10. The composition according to one of the preceding claims 2 to 9 **characterised in that** the at least one O/W emulsifier is selected from the group consisting of ethoxylated primary or secondary C₁₀-C₂₀ alcohols, especially primary or secondary C₁₁-C₁₅ alcohols, anionic sulfosuccinates and mixtures thereof.

11. The composition according to Claim 10 **characterised in that** the ethoxylated alcohol is ethoxylated with an average of 1 to 30, preferably 5 to 20 and especially with about 9 moles ethylene oxide.

12. The composition according to one of the preceding claims **characterised in that** the at least one fatty acid ester of the oil phase is selected from the group consisting of the esters of C₁-C₃ alcohols with saturated C₁₀-C₂₂ fatty acids.

13. The composition according to one of claims 1 to 11 **characterised in that** the at least one fatty acid ester is selected from the group consisting of esters of methanol, ethanol, propanol, isopropanol, butanol, butan-2-ol, tert.-butyl alcohol with undecanoic acid, lauric acid, tridecanoic acid, myristic acid, pentadecanoic acid or palmitic acid.

14. The composition according to Claim 13 **characterised in that** the fatty acid ester is isopropyl myristate.

15. The composition according to one of the preceding claims comprising
(a) 0.5 wt.% to 10 wt.% W/O emulsifier,
(b) 0.5 wt.% to 10 wt.% O/W emulsifier,
(e) 20 to 70 wt.% anionic polyelectrolyte and
(f) at least 15 wt.% fatty acid ester.

16. A process for manufacturing the composition according to one of claims 1 to 15 in which the at least one W/O emulsifier and the at least one fatty acid ester are blended together as the oil phase, the AMPS salt and acrylic acid monomers in partially neutralised form including crosslinking agent are separately blended together in the aqueous phase, the aqueous phase is then emulsified in the oil phase, the monomer mixture is then polymerised and finally the at least one O/W emulsifier is optionally added.

17. Use of the composition according to claims 1 to 15 or manufactured according to Claim 16 as a thickener, especially as a thickener for cosmetic or pharmaceutical products, preferably a rinse-off product such as a shampoo.

## Revendications

1. Composition contenant une phase huileuse, une phase aqueuse et au moins un agent émulsionnant de type eau-dans-l'huile (agent émulsionnant E/H), dans laquelle la phase aqueuse contient au moins un polyélectrolyte anionique ramifié ou réticulé, qui peut être fabriqué par copolymérisation d'un sel de l'acide 2-acrylamido-2-méthylpropanesulfonique (sel d'AMPS) avec un acide acrylique partiellement neutralisé, et la phase huileuse est constituée d'au moins un ester d'un alcool en C₁-C₅ et d'un acide gras en C₁₀-C₂₂ saturé ou insaturé.

2. Composition selon la revendication 1, **caractérisée en ce qu'**elle contient en outre au moins un agent émulsionnant huile-dans-l'eau (agent émulsionnant H/E).

3. Composition selon la revendication 1 ou selon la revendication 2, **caractérisée en ce que** le sel de l'acide 2-acrylamido-2-méthylpropanesulfonique est le sel de sodium.

4. Composition selon l'une quelconque des revendications précédentes, **caractérisée en ce que** l'acide acrylique est neutralisé avec de la soude caustique.

5. Composition selon l'une quelconque des revendications précédentes, **caractérisée en ce que** le au moins un agent émulsionnant E/H est un dérivé de l'acide succinique choisi dans le groupe constitué d'esters, de semi-esters, de sels, de semi-sels d'acide succinique contenant de l'azote et à substitution polyisobutényle, de formes mixtes de ces composés et de mélanges de ceux-ci.

6. Composition selon la revendication 5, **caractérisée en ce que** le radical polyisobutényle présente un poids moléculaire moyen de 1000 (moyenne en nombre).

7. Composition selon la revendication 5 ou selon la revendication 6, **caractérisée en ce que** l'azote est présent dans le ou les radicaux ester et/ou dans les contre-ions formant le sel/semi-sel.

8. Composition selon la revendication 5 ou selon la revendication 6, **caractérisée en ce que** le dérivé d'acide succinique est un semi-sel/semi-ester de l'acide succinique, dans laquelle le radical ester ou le contre-ion donnant le semi-sel est choisi du groupe constitué de la triéthylamine et du diéthylaminoéthanol.

9. Composition selon la revendication 5, **caractérisée en ce que** l'agent émulsionnant E/H est représenté par la substance hydroxyethyldiethonium polyisobutenyl triethylaminosuccinate selon la désignation INCI.

10. Composition selon l'une quelconque des revendications précédentes 2 à 9, **caractérisée en ce que** le au moins un agent émulsionnant H/E est choisi dans le groupe constitué d'alcools en C₁₀-C₂₀ primaires ou secondaires éthoxylés, en particulier d'alcools en C₁₁-C₁₅ primaires ou secondaires, de sulfosuccinates anioniques et de leurs mélanges.

11. Composition selon la revendication 10, **caractérisée en ce que** l'alcool éthoxylé est éthoxylé avec, en moyenne, 1 à 30, de préférence 5 à 20 et, en particulier, environ 9 moles d'oxyde d'éthylène.

12. Composition selon l'une quelconque des revendications précédentes, **caractérisée en ce que** le au moins un ester d'acide gras de la phase huileuse est choisi dans le groupe constitué des esters d'alcools en C₁-C₃ et d'acides gras en C₁₀-C₂₂ saturés.

13. Composition selon l'une quelconque des revendications 1 à 11, **caractérisée en ce que** le au moins un ester d'acide gras est choisi dans le groupe constitué d'esters de méthanol, d'éthanol, de propanol, d'isopropanol, de butanol, de butan-2-ol, d'alcool de tert-butyle et d'acide undécanoïque, d'acide laurique, d'acide tridécanoïque, d'acide myristique, d'acide pentadécanoïque ou d'acide palmitique.

14. Composition selon la revendication 13, **caractérisée en ce que** l'ester d'acide gras est le myristate d'isopropyle.

15. Composition selon l'une quelconque des revendications précédentes 2 à 14, **caractérisée en ce qu'**elle contient :
(a) 0,5 % en poids à 10 % en poids d'agent émulsionnant E/H,
(b) 0,5 % en poids à 10 % en poids d'agent émulsionnant H/E,
(e) 20 à 70 % en poids d'un polyélectrolyte anionique, et
(f) au moins 15% en poids d'un ester d'acide gras.

16. Procédé de fabrication de la composition selon l'une quelconque des revendications 1 à 15, dans lequel on mélange l'un à l'autre le au moins un agent émulsionnant E/H et le au moins un ester d'acide gras comme phase huileuse, on mélange séparément l'un à l'autre les monomères de sel d'AMPS et d'acide acrylique sous forme partiellement neutralisée, y compris un agent de réticulation, comme phase aqueuse, on émulsionne ensuite la phase aqueuse dans la phase huileuse, puis on polymérise le mélange de monomères et, en fin de compte, on ajoute éventuellement le au moins un agent émulsionnant H/E.

17. Utilisation de la composition selon les revendications 1 à 15 ou fabriquée selon la revendication 16, comme agent épaississant, en particulier, comme agent épaississant pour des produits cosmétiques ou pharmaceutiques, de préférence, pour un produit de lavage, tel qu'un shampooing.
